**Europäisches Patentamt**

**European Patent Office**

**Office européen des brevets**

(19)

(11) Veröffentlichungsnummer: **0 144 820**
**B1**

(12) **EUROPÄISCHE PATENTSCHRIFT**

(45) Veröffentlichungstag der Patentschrift:
26.07.89

(21) Anmeldenummer: **84113705.2**

(22) Anmeldetag: **13.11.84**

(51) Int. Cl.⁴: **G 01 N 31/22, G 01 N 33/52**

(54) **Verfahren zum Nachweis von allergenhaltigem Hausstaub.**

(30) Priorität: **06.12.83 DE 3344087**

(43) Veröffentlichungstag der Anmeldung:
**19.06.85 Patentblatt 85/25**

(45) Bekanntmachung des Hinweises auf die Patenterteilung:
**26.07.89 Patentblatt 89/30**

(84) Benannte Vertragsstaaten:
**AT BE CH FR GB IT LI LU NL SE**

(56) Entgegenhaltungen:
**Keine**

(73) Patentinhaber: **Werner & Mertz GmbH,
Ingelheimstrasse 1-3, D-6500 Mainz 1 (DE)**

(72) Erfinder: **Bischoff, Edelbert, Dr., Leibnizstrasse 52,
D-6719 Kirchheim-Bolanden (DE)**
Erfinder: **Schirmacher, Wolfgang, Dipl.-Chem.,
Rilkeallee 28, D-6500 Mainz 31 (DE)**

(74) Vertreter: **Zumstein, Fritz jun., Dr. et al, Dr. F. Zumstein
sen. Dr. E. Assmann Dr. R. Koenigsberger Dipl.-Ing. F.
Klingseisen Dr. F. Zumstein jun. Bräuhausstrasse 4,
D-8000 München 2 (DE)**

ACTORUM AG

## Beschreibung

Dem Auftreten von Hausstaubmilben (Dermatophagoides pteronyssinus, farinae etc.) wird in neuerer Zeit zunehmend Aufmerksamkeit gewidmet (vgl. Zitat 1). Grund dafür ist, daß festgestellt wurde, daß ein beträchtlicher Teil der Bevölkerung – man rechnet mit etwa 5% – unter asthmatischen Erscheinungen leidet, die auf Bestandteile des Hausstaubes zurückzuführen sind.

Bei diesen Bestandteilen handelt es sich hauptsächlich um den Kot der obengenannten Hausstaubmilben sowie um Bruchstücke abgestorbener Milben. Im Darmtrakt der lebenden und abgestorbenen Milben sind die die Gesundheitsstörungen auslösenden Allergene enthalten (vgl. Zitat 2). Eine lebende Milbe selbst enthält nur etwa ein Tausendstel der Allergenmenge, die sie in der Zeit ihres Lebens erzeugt. Der Kot selbst ist vielfach von Pilzen besiedelt, die ihrerseits ebenfalls Allergene hervorbringen können. Die Hauptmenge der Allergene befindet sich in den Staubablagerungen, und zwar auch dann noch, wenn die ursprüngliche Milbenpopulation durch Bekämpfungsmaßnahmen abgetötet bzw. reduziert wurde.

Aufenthaltsorte der Hausstaubmilben sind Polstermöbel, Betten, Fußböden und Wände, insbesondere wenn sie mit textilen Materialien überzogen sind. Flächenmäßig gesehen dürften Teppichböden die größte Rolle spielen.

Von den Fußböden und Polstermöbeln wird durch Begehen und Benutzen Feinststaub aufgewirbelt, der sich oft an unzugänglichen Stellen, die weitgehend frei von Luftzug sind, z.B. hinter Schränken, ansammelt. Auch beim Staubsaugen selbst wird dieser Feinststaub zum Teil aufgewirbelt und gelangt so auch an Stellen, die selbst nicht von Milben besiedelt sind. In allen Fällen führt erneutes Aufwirbeln zu erneuten Belästigungen der Bewohner der betreffenden Räume.

Die Beseitigung der Ursachen für die Hausstaub-Allergie hat zur Voraussetzung, daß die Ursache erkannt wird. Denn es gibt auch andere Allergien, wie z.B. gegen Pollen (Heuschnupfen). Die Hausstaub-Allergie belästigt jedoch die davon betroffenen Menschen das ganze Jahr über, da Milbenkot und Milbenreste praktisch ununterbrochen vorhanden sind. Arzt und Patient stehen deshalb immer wieder vor der Frage, ob ein Milbenbefall vorliegt.

Aufgrund des derzeitigen Standes der Technik gibt es als Nachweismethode nur das mikroskopische Erkennen der Milben selbst. Dieses Verfahren ist jedoch mit so großen Schwierigkeiten verbunden, daß es nur von wenigen darauf spezialisierten Fachlaboratorien durchgeführt werden kann. Dazu sind die Entnahme der Staubprobe, der Versand derselben unter vorgeschriebenen Bedingungen und dann die Untersuchung im Laboratorium notwendig. Da die Milben neben dem sonstigen Schmutz nicht zu erkennen sind, müssen sie von der Staubprobe abgetrennt und dann identifiziert werden.

Das bewährteste bekannte Abtrennverfahren besteht in der Flotation, verbunden mit dem manuellen Isolieren der aufschwimmenden Tiere sowie deren Identifikation unter dem Mikroskop (vgl. Zitat 3). Abgesehen von der Tatsache, daß dies nur erfahrene Fachleute durchführen können, ist hervorzuheben, daß ein beträchtlicher Zeitaufwand erforderlich ist, um eine Probe zu bearbeiten; hinzukommen der Versand und das Risiko der Proben-Veränderung während des Transportes.

Allen Identifikationsverfahren, die vom Vorhandensein der Milben selbst ausgehen, haftet der Nachteil an, daß Aussagen über die Belastung des Staubes durch Allergene, die die eigentliche Krankheitsursache darstellen, nicht zu erhalten sind.

Staubproben, insbesondere solche feinster Struktur, wie sie sich nach dem Aufwirbeln aus der Luft ablagern, können besonders reich an Allergenen sein und wenige oder gar keine Milben enthalten.

Aufgabe der vorliegenden Erfindung war es daher, ein einfaches und zuverlässiges Nachweisverfahren zum Erkennen von allergenhaltigem Hausstaub zu schaffen.

Erfindungsgemäß wurde nun festgestellt, daß ein (wesentlich einfacherer) Nachweis geführt werden kann, der charakteristisch für die Belastung des Hausstaubes mit Ausscheidungsprodukten der Hausstaubmilben ist, unabhängig davon, ob nun lebende bzw. tote Milben im Moment des Nachweises vorhanden sind oder nicht.

Der Nachweis knüpft an Bestandteile der Ausscheidungsprodukte der Milben an, wie sie sich sowohl im Verdauungstrakt der Milben selbst als auch in deren Exkrementen befinden.

Dieser Nachweis ist problemlos sowohl von Fachleuten als auch Laien durchführbar und gibt nicht nur eine Aussage über die Anwesenheit von Exkrementen der Hausstaubmilben im Hausstaub, sondern ermöglicht auch eine Beurteilung des momentanen bzw. früheren Befalles mit Milben.

Das erfindungsgemäße Nachweisverfahren zur Feststellung des Vorkommens von allergenhaltigem Hausstaub, wie er sich bei oder nach dem Auftreten von Hausstaubmilben ergibt, besteht nun darin, daß man Proben von vorkommendem Staub, wie er in Gebäuden anfällt, in wäßrig-alkoholischer Alkalimetallhydroxid-Lösung gründlich suspendiert und mit dem entstehenden Extrakt eine Farbreaktion mit Hilfe einer aromatischen Diazoverbindung durchführt, wobei die Farbreaktion hinsichtlich ihres Auftretens als Indiz für die Belastung des Staubes mit Hausstaubmilben-Rückständen bewertet wird.

Im einzelnen ist für die Durchführung des erfindungsgemäßen Nachweisverfahrens folgendes zu bemerken. Als Alkohol für die eingesetzte wäßrigalkoholische Lösung verwendet man bevorzugt einen mit Wasser unbegrenzt mischbaren Alkohol, wie z.B. niedere einwertige aliphatische Alkohole. Dabei ist Methanol besonders bevorzugt; aber auch Äthanol kann eingesetzt werden. Das Gewichtsverhältnis von Alkohol zu Wasser in der wäßrig-alkoholischen Lösung kann variieren und beträgt im allgemeinen (10 bis 95):(90 bis 5). Besonders bewährt hat sich ein Gewichtsverhältnis von Alkohol zu Wasser von (50 bis 90):(50 bis 10), speziell von (75 bis 90):(25 bis 10).

Das in der wäßrig-alkoholischen Lösung eingesetzte Alkalimetallhydroxid ist bevorzugt Kaliumhydroxid und Natriumhydroxid. Aber auch Lithiumhydroxid kann eingesetzt werden. Die Menge des Alkalimetallhydroxids in der wäßrig-alkoholischen Lösung kann variieren; im allgemeinen beträgt sie 0,1 bis 50 Gew.-%. Vorteilhaft ist ein Alkalimetallhydroxidgehalt von 0,1 bis 20 Gew.-%, bevorzugt 0,1 bis 10 Gew.-%. Ganz speziell bevorzugt beträgt der Alkalimetallhydroxidgehalt in der wäßrig-alkoholischen Lösung etwa 4 bis 5 Gew.-%.

Für die Farbreaktion eignen sich grundsätzlich alle Verbindungen, die zusammen mit dem Milbenrückstände enthaltenden Staub Farbreaktionen ergeben. Die im vorliegenden Fall angewandte Reaktion beruht auf der Fähigkeit der Milbenrückstände (Milbenkotreste und Bruchstücke abgestorbener Milben), mit aromatischen Diazoverbindungen eine Farbreaktion zu ergeben. Als aromatische Diazoverbindung ist daher jede aromatische Diazoverbindung geeignet, die mit der Extraktionslösung (im Falle des Vorhandenseins von Hausstaubmilbenrückständen) eine Farbreaktion eingeht, deren resultierender Farbstoff in der Extraktionslösung löslich ist. Als aromatische Diazoverbindung besonders bewährt hat sich Diazosulfanilsäure (4-Diazobenzosulfonsäure), die eine ziegelrote Färbung ergibt. Die aromatische Diazoverbindung kann in fester (gegebenenfalls in üblicher Weise stabilisierter) Form oder in Form einer wäßrigen, insbesondere gesättigten Lösung eingesetzt werden.

Bei dem erfindungsgemäßen Nachweisverfahren geht man zweckmäßig in der Weise vor, daß man eine gewonnene Staubprobe (nach Entfernung der Grobanteile) mit der wäßrig-alkoholischen Alkalimetallhydroxid-Lösung durchtränkt. Dabei kann das Volumenverhältnis der Staubprobe zur Extraktionslösung variieren. Im allgemeinen beträgt dieses Volumenverhältnis der Staubprobe zur Extraktionslösung (0,1 bis 2):(1). Besonders bewährt hat sich ein Volumenverhältnis von 0,5:1.

Die mit der Extraktionslösung versetzte Staubprobe wird mit einer aromatischen Diazoverbindung, insbesondere Diazosulfanilsäure (4-Diazobenzolsulfonsäure) versetzt. Dies erfolgt z.B. durch Zugabe der aromatischen Diazoverbindung in fester, gegebenenfalls stabilisierter Form. Stattdessen kann die aromatische Diazoverbindung aber auch in gelöster Form, insbesondere als gesättigte wäßrige Lösung (einige Tropfen) zudosiert werden. Das Ganze wird sodann durch Schütteln oder Rühren gut vermischt. Die erhaltene Extraktsuspension läßt man stehen, wobei sich der Staub im allgemeinen von der Extraktionslösung absetzt (wenige Minuten).

Aufgrund der vorhandenen aromatischen Diazoverbindung, z.B. Diazosulfanilsäure, bildet sich bei Anwesenheit von Milbenexkrementen eine ziegelrote Färbung, die, wie die Anmelderin festgestellt hat, in ihrer Intensität proportional zu der in der Staubprobe vorhandenen Milbenkotkonzentration ist.

Zur Verdeutlichung der Farberkennung kann man die (über dem sedimentierten Staub) überstehende Extraktionslösung abtrennen und die Farbintensität in dieser Form beurteilen. Man kann aber auch einen kleinen Streifen weißes Filterpapier von definierter Größe (z.B. 0,5 cm²) in die Farblösung eintauchen und die auf dem Papier verbleibende Färbung bewerten.

Dem erfindungsgemäßen Nachweisverfahren selbst liegen einige spezielle und zum Teil überraschende Befunde zugrunde:

1) Um die für den Farbnachweis relevanten Kotbestandteile bei Normaltemperatur mit der wäßrig-alkoholischen Extraktionslösung schnell und erschöpfend zu extrahieren, ist eine Alkalimetallhydroxid-Lösung (z.B. von KOH oder von NaOH) in der angegebenen Konzentration nötig (eine entsprechend konzentrierte Sodalösung, wie sie eigentlich gemäß der nachfolgenden Ziffer 2) nötig wäre, führt nicht zum Erfolg).

2) Die Farbreaktion mit der aromatischen Diazoverbindung, insbesondere mit Diazosulfanilsäure, würde an sich am besten in nur wäßriger, sodaalkalischer Lösung verlaufen. (In nur wäßriger Alkalimetallhydroxid-Lösung, z.B. von 4–5 Gew.-% KOH oder NaOH, findet die gewünschte Farbreaktion – wie die Anmelderin festgestellt hat – nicht statt.) Ausgehend von dieser besonderen Problematik im Hinblick auf die Gestaltung eines einfachen Nachweisverfahrens, wurde nun aber überraschend gefunden, daß bei Einsatz einer Extraktionslösung von Alkalimetallhydroxid in einem Lösungsmittelgemisch von Alkohol und Wasser, insbesondere in einem Gemisch, bei dem das Gewichtsverhältnis von Alkohol zu Wasser (50 bis 90):(50 bis 10) beträgt, einerseits die für den Farbnachweis relevanten Kotbestandteile praktisch quantitativ extrahiert werden, und daß andererseits in derselben Extraktionslösung die gewünschte Farbreaktion mit der aromatischen Diazoverbindung, insbesondere Diazosulfanilsäure, stattfindet. Außerdem verbessert der Alkohol die Benetzung der Staubproben (und bewirkt gleichzeitig eine schnelle und gleichmäßige Farbverteilung auf dem gegebenenfalls eingetauchten Filterpapier).

Nachstehend werden weitere Einzelheiten des erfindungsgemäßen Nachweisverfahrens angegeben. Das gefundene Analysenverfahren auf Befall von Hausstaub mit allergenhaltigen Rückständen von Hausstaubmilben läßt sich mannigfaltig ausgestalten. Dies gilt sowohl für die Probenentnahme des Staubes, als auch für das Zusammenbringen des Staubes mit der Extraktionslösung, als auch für den Ablauf der Farbreaktion.

Im Sinne einer besonders effizienten Staubgewinnung kann in die Ansaugröhre eines üblichen Staubsaugers ein Filter (insb. Stoff- oder Papier-Filter) eingelegt werden, z.B. in Form eines kleines Tuches (insbesondere Taschentuches). Es hat den Vorteil, daß der Feinstaub gezielt und konzentriert gesammelt wird. Bei der Probenentnahme selbst ist zu berücksichtigen, daß Bodenflächen, die des öfteren abgesaugt werden, eine für den Nachweis ausreichende Staubmenge eventuell nicht mehr enthalten können; in diesem Falle führt man dann zweckmäßig das Absaugen an weniger zugänglichen Stellen durch. Besonders vorteilhaft erfolgt die Staubgewinnung durch Absaugen von Polstermöbeln und Betten.

Für die Extraktion der Staubproben eignen sich besonders kleine verschließbare Glas- und Kunststoffbehälter mit einem Fassungsvermögen von einigen Millilitern (z.B. 1 bis 10 Milliliter), die mit Markierungen zur Volumendosierung der Staubprobe und der Extraktionslösung versehen sind. In speziellen Fällen kann die Extraktion der Staubprobe aber auch z.B. auf einem Uhrglas erfolgen.

Die Effizienz des erfindungsgemäßen Verfahrens wird durch den folgenden Vergleich illustriert:

An 20 Staubproben, die von Ärzten aus verschiedenen Häusern mit Verdacht auf Hausstaub-Allergie eingesandt wurden, erfolgte zunächst eine biologische Beurteilung unter Anwendung des Flotationsverfahrens (gemäß Stand der Technik, wie oben beschrieben). Diese Bewertung der Proben ergab einen mittelstarken, schwachen bzw. keinen Befall.

Danach wurden mit den gleichen Proben erfindungsgemäße Bewertungen durchgeführt, und zwar von einer Person, die keine Kenntnis von der (vorherigen) biologischen Beurteilung hatte. Die erfindungsgemäßen Farbreaktionen zeigten mittleren, schwachen und keinen Befall. Beim Vergleich dieser Ergebnisse mit dem Laborjournal, in dem die (vorherigen) biologischen Befunde registriert waren, ergab sich völlige Übereinstimmung.

Beispiel 1

Man versieht einen normalen Staubsauger mit einem neuen Staubbeutel und saugt den zu untersuchenden Textilbereich (z.B. Teppiche, Polstermöbel, Deckbetten, Matratzen) gründlich ab. Nach Beendigung des Absaugens entleert man den gesamten Staubbeutelinhalt auf ein Blatt Papier und entfernt durch leichtes Abklopfen und Abheben die Grobanteile. Der auf dem Papier verbleibende Feinstaub wird für den Farbnachweis verwendet.

Für den Farbnachweis dosiert man die Feinstaubprobe (ca. 2 ml) in einen Glas- oder Kunststoffzylinder von ca. 8 ml Inhalt (Durchmesser ca. 1,5 cm). Dazu dosiert man ca. 4 ml einer wäßrigmethanolischen Extraktionslösung, bei der das Gewichtsverhältnis von Methanol zu Wasser 75:25 beträgt und in der 5 Gew.-% KOH gelöst sind. Dazu fügt man die erforderliche Menge Diazosulfanilsäure (1 Mikrospatelspitze; ca. 20 mg/ml Staub) und schüttelt das verschlossene Gefäß ca. 10 Sekunden. Danach öffnet man das Gefäß (Gasentwicklung). Nach etwa 3 Minuten taucht man einen Streifen aus weißem Filterpapier (0,5 cm²) in die abgesetzte Extraktionslösung und beurteilt die bei Milbenbefall auftretende ziegelrote Färbung (diese bleibt bei Abwesenheit von Milbenrückständen aus).

Beispiel 2

Die Staubprobenahme erfolgt analog dem Beispiel 1.

Man dosiert die Feinstaubprobe (ca. 0,5 ml) auf ein Uhrglas von ca. 6 cm Durchmesser, fügt 10 Tropfen Extraktionslösung (Methanol/Wasser-Gemisch im Gewichtsverhältnis 90:10, worin 4 Gew.-% NaOH gelöst sind) und 10 Tropfen gesättigte, wäßrige Diazosulfanilsäure-Lösung hinzu. Nach kurzzeitigem Durchmischen drückt man einen Streifen weißes Filterpapier von definierter Größe (0,5 cm²) einseitig gegen die breiige Masse. Bei Milbenbefall zeigt sich eine ziegelrote Färbung, die man (vorteilhaft) über die unverschmutzte Papierseite beurteilt.

Zitat 1: G. Hoffmann in «Luftqualität in Innenräumen», Gustav Fischer Verlag, Stuttgart – New York 1982, Seite 385 bis 401

Zitat 2: E. Petri et al in Allergologie, Jg. 5, Nr. 3/1982, Seite 109 bis 119

Zitat 3: J. E. M. H. van Bronswijk et al in Allergie und Immunol. 24 (1978), Seite 18 bis 28

**Patentansprüche**

1. Verfahren zur Feststellung des Vorkommens von allergenhaltigem Hausstaub, wie er sich bei oder nach Auftreten von Hausstaubmilben ergibt, dadurch gekennzeichnet, daß man Proben von in Gebäuden vorkommendem Staub in wäßrig-alkoholischer Alkalimetallhydroxid-Lösung gründlich suspendiert und mit dem entstehenden Extrakt eine Farbreaktion mit Hilfe einer aromatischen Diazoverbindung durchführt, wobei die Farbreaktion hinsichtlich ihres Auftretens als Indiz für die Belastung des Staubes mit Hausstaubmilben-Rückständen bewertet wird.

2. Verfahren nach Anspruch 1, dadurch gekennzeichnet, daß der Alkalimetallhydroxid-Gehalt in der wäßrig-alkoholischen Lösung 0,1 bis 50 Gew.-% beträgt.

3. Verfahren nach einem der vorhergehenden Ansprüche, dadurch gekennzeichnet, daß das Gewichtsverhältnis von Alkohol zu Wasser in der Extraktionslösung (10 bis 95):(90 bis 5) beträgt.

4. Verfahren nach einem der vorhergehenden Ansprüche, dadurch gekennzeichnet, daß das Volumenverhältnis der Staubprobe zur Extraktionslösung (0,1 bis 2):1 beträgt.

5. Verfahren nach einem der vorhergehenden Ansprüche, dadurch gekennzeichnet, daß eine aromatische Diazoverbindung eingesetzt wird, deren resultierender Farbstoff in der Extraktionslösung löslich ist.

6. Verfahren nach einem der vorhergehenden Ansprüche, dadurch gekennzeichnet, daß eine aufgetretene Farbreaktion hinsichtlich der Intensität der Färbung mit einer standardisierten Farbskala verglichen und aufgrund dieses Vergleichs die Konzentration an Milbenrückständen beurteilt wird.

**Claims**

1. A process for detecting the incidence of domestic dust containing allergens, as it occurs when domestic dust mites are or were present, characterized in that samples of dust, present in buildings, are completely suspended in an aqueous alcohol solution of alkali metal hydroxide and in that a colour-differing reaction is carried out with the resultant extract, by means of an aromatic diazo compound, and the colour reaction, if it occurs, being taken as evidence that the dust contains residue of domestic dust mites.

2. A process according to claim 1, characterized in that the aqueous alcohol solution contains 0.1 to 50% by weight of alkali metal hydroxide.

3. A process according to any one of the preceding claims, characterized in that the ratio by weight of alcohol to water in the extracting solution is (10 to 95):(90 to 5).

4. A process according to any one of the preceding claims, characterized in that the ratio by volume of the dust sample to the extracting solution is (0.1 to 2):1.

5. A process according to any one of the preceding claims, characterized in that an aromatic diazo compound is used, the resultant colour thereof being soluble in the extracting solution.

6. A process according to any one of the preceding claims, characterized in that the colour of any colour-differing reaction occurring is compared, in respect of its colour intensity, with a standardized colour chart and that the concentration of mite residue is assessed on the basis of this comparison.

Revendications

1. Procédé pour la constatation de la présence de poussière domestique contenant des allergènes, telle qu'elle se présente lors de l'apparition d'acariens de poussière domestique ou après, caractérisé en ce qu'on met totalement en suspension en solution hydroalcoolique d'hydroxyde de métal alcalin des échantillons de poussière présente dans des bâtiments et on effectue, avec l'extrait résultant, une réaction colorée à l'aide d'un composé diazo aromatique, la réaction colorée étant estimée en ce qui concerne son apparition en tant qu'indice de la charge de la poussière en résidus d'acariens de poussière domestique.

2. Procédé selon la revendication 1, caractérisé en ce que la teneur en hydroxyde de métal alcalin dans la solution hydroalcoolique est de 0,1 à 50% en poids.

3. Procédé selon l'une des revendications précédentes, caractérisé en ce que le rapport en poids de l'alcool à l'eau dans la solution d'extraction est de (10 à 95):(90 à 5).

4. Procédé selon l'une des revendications précédentes, caractérisé en ce que le rapport en volume de l'échantillon de poussière à la solution d'extraction est de (0,1 à 2):1.

5. Procédé selon l'une des revendications précédentes, caractérisé en ce que l'on met en œuvre un composé diazo aromatique dont la matière colorante résultante est soluble dans la solution d'extraction.

6. Procédé selon l'une des revendications précédentes, caractérisé en ce qu'une réaction colorée qui a lieu est comparée en ce qui concerne l'intensité de la coloration avec une échelle de couleur standardisée et la concentration en résidus d'acariens est estimée sur la base de cette comparaison.